# EUROPEAN PATENT SPECIFICATION

(11) **EP 1 599 024 B1**
(45) Date of publication and mention of the grant of the patent: **25.07.2007**
(21) Application number: 05076636.9
(22) Date of filing: 14.05.2002
(51) Int. Cl.: H04M 1/725, G07F 11/00

(54) **Portable communication device for use in a vending system**
Tragbares Kommunikationsgerät zum Gebrauch in einem Verkaufssystem
Appareil de communication portable pour usage dans un système de vente

(30) Priority: 14.05.2001 GB 0111722; 10.12.2001 GB 0129491; 10.12.2001 GB 0129490; 10.04.2002 GB 0208275
(43) Date of publication of application: 23.11.2005
(62) Divisional of application: 02769514.7
(73) Proprietor: Innovision Research & Technology PLC, Cirencester, Gloucestershire GL7 1RQ (GB)
(72) Inventor: White, Andrew, Innovision Res. & Technology PLC, Cirencester, Gloucestershire GL7 1RQ (GB); Borrett, Marc Adrian, Innovision Res. & Tech. PLC, Cirencester, Gloucestershire GL7 1RQ (GB); Mapleston, David B., Innovision Res. & Tech. PLC, Cirencester, Gloucestershire GL7 1RQ (GB)
(74) Representative: Clark, Jane Anne

(56) References cited:
- WO-A-00/52655
- WO-A-00/77697
- WO-A-01/20844
- GB-A- 2 327 565
- GB-A- 2 344 025

## Description

This invention relates to a vending system, a vending device for use in a vending system and a portable communications device for use in a vending system, especially a portable communications device such as a telephone arranged to operate using a mobile telecommunications network, and a payment system.

Telephones that operate using a mobile telecommunications network are variously known as mobile telephones, cellular telephones and cellphones. For simplicity, the term "mobile telephone" will be used hereinafter.

WO00/52655 discusses a vending machine with a transponder interrogator. A customer carries a transponder embedded within an ornamental item or useful item such as a key ring and when the customer desires to make a purchase from the vending machine, the customer presents the transponder to the transponder interrogator. The transponder is identified and the purchase price is debited from a credit amount associated with the transponder.

WO00/77697 discusses a method and system for remote payments utilising a cellular telephone in which a user calls the cellular telephone number of a vending machine and a telephone connection is established between the vending machine and the cellular telephone for the exchange of identification information to determine whether a desired transaction is available. If so, the vending machine charges the transaction to the cellular telephone.

In one aspect, the present invention provides a vending system as set out in claim 1. In another aspect, the present invention provides a vending device as set out in claim 12. In another aspect, the present invention provides a portable communications device as set out in claim 16. In another aspect, the present invention provides a payment system as set out in claim 18.

As used herein the term "passive data storage device" means a device that is not self-powered but that derives power from the main body when the fascia is attached to the main body.

As used herein the term passive detachable element means an element that is not self-powered and that, if it requires a power supply, derives it from the main body when the fascia is attached to the main body.

The use of a data storage device that does not require its own power supply enables costs to be kept down and, moreover, because such passive data storage devices are relatively cheap to produce and can be incorporated into products relatively easily (for example during a plastics moulding process), the incorporation of the passive data storage device does not add significantly to costs.

Embodiments of the present invention will now be described, by way of example, with reference to the accompanying drawings, in which:
Figure 1 shows a diagrammatic perspective view of a mobile telephone with a removable fascia separated from a main body of the mobile telephone;
Figure 2 shows a functional block diagram of functional components of a mobile telephone, including a reader unit for enabling detection of a replacement fascia;
Figure 3 shows a functional block diagram of an example of the reader unit shown in Figure 2;
Figure 4 shows a functional block diagram of an example of a passive data storage device;
Figure 5 shows a more detailed functional block diagram of an example of the reader unit;
Figure 6 shows a more detailed functional block diagram of one example of a passive data storage device;
Figure 7 shows a functional block diagram of another example of a passive data storage device;
Figure 8 shows a block diagram illustrating an example of a portable communication device not falling within the scope of the claims;
Figure 9 shows a functional block diagram of computing apparatus such as a personal digital assistant;
Figure 10 shows a very diagrammatic representation of an electronic device in the form of a power tool having a number of replaceable heads, two of which are shown, one attached to the power tool;
Figure 11 shows a functional block diagram of functional components of an electrical appliance embodying the invention, including a reader unit for enabling detection of a replacement fascia; and
Figure 12 shows an example of a vending system.

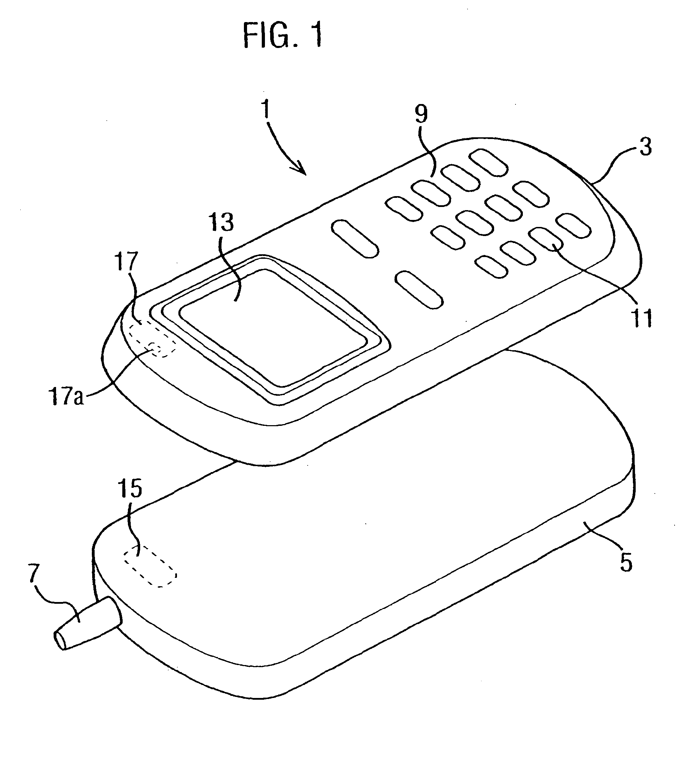

Figure 1 illustrates a typical mobile telephone or cellphone 1 comprising a main body 5 and an attachable, and in this case also removable, fascia 3. The fascia 3 is formed with a user input array 9 which comprises a plurality of holes 11 for engagement with key pads (not shown) provided on the main body 5 of the mobile telephone 1. The fascia 3 is also provided with, in this example, a transparent plastics window 13 through which a liquid crystal display (LCD) (not shown in Figure 1) provided on the main body 5 of the mobile telephone 1 can be viewed. The mobile telephone 1 also comprises an aerial 7 which allows telecommunication signals to be transmitted and received.

Mounted upon, or embedded within, an inside surface of the removable fascia 3 is a passive data storage device 17. Generally, the fascia 3 will be moulded from a plastics material and the passive data storage device 17 encapsulated in a housing fitted to (for example embedded in or mounted to the surface of) the fascia 3 during the moulding process.

The main body 5 of the mobile telephone 1 carries a reader unit 15. The reader unit 15 is positioned such that, when the fascia 3 is fitted to the main body 5, the passive data storage device 17 will be in range of, as shown will lie adjacent to, the reader unit 15 so that, as will be described in detail below, couplers of the passive data storage device 17 and reader unit 15 couple to enable the passive data storage device 17 to derive a power supply from a signal supplied by the reader unit 15 and, when so activated, to transmit control data contained in its memory.

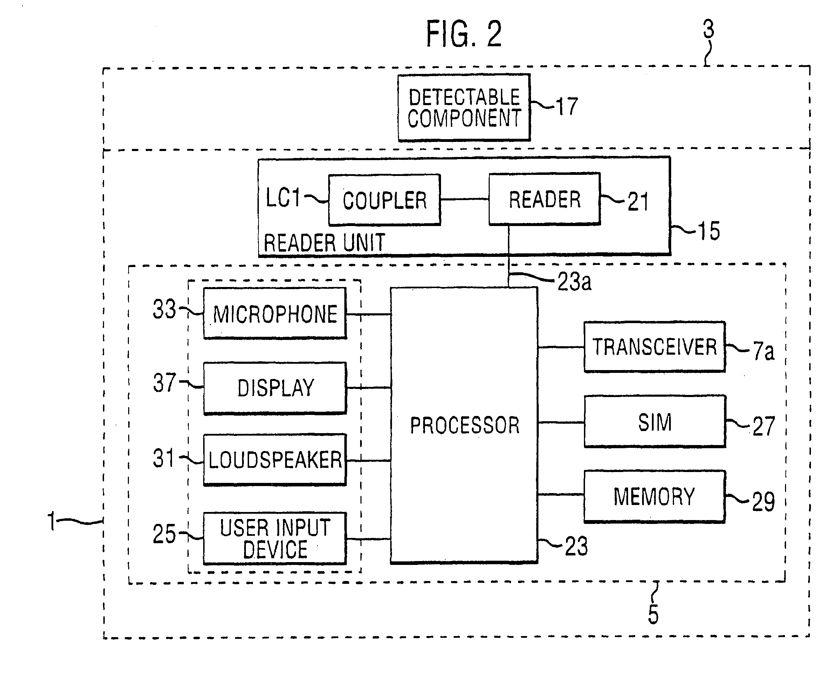

Figure 2 shows a functional block diagram of the mobile telephone 1. The main body 5 of the mobile telephone 1 comprises: a microphone 33 and a loudspeaker 31 enabling a user to input speech and hear audio output respectively; a user input device 25 (in this case a keyboard) enabling the user to input numbers to be called, other information and instructions for controlling various features of the mobile telephone 1; a display 37 on which incoming or outgoing telephone numbers, SMS text messages and other information can be displayed; and a transceiver 7a which with the aerial 7 shown in Figure 1 enables transmission and reception of communications over a mobile telecommunications network using, for example, the GSM (Global System for Mobile Communications), GPRS (General Packet Radio Service) or 3G (Third Generation, GSM) system, or future such networks.

The above functional components are coupled, via appropriate interfaces (not shown) to a mobile telephone processor 23 which controls the overall operation of the mobile telephone 1. The mobile telephone processor 23 is associated with a non-volatile memory 29 which has a read-only portion which contains control operation software data and information data and a writable portion that, for example, enables storage of telephone numbers and messages input to the mobile telephone by the user or received by the mobile telephone over the mobile telecommunications network.

As is known in the art, the mobile telephone 1 further comprises a subscriber identification module (SIM) 27. The SIM 27 is a detachable module which provides user specific data and also algorithms and data specific to the operator of the mobile telecommunications service provider. The mobile telephone processor 23 is coupled to the reader unit 15 via line 23a.

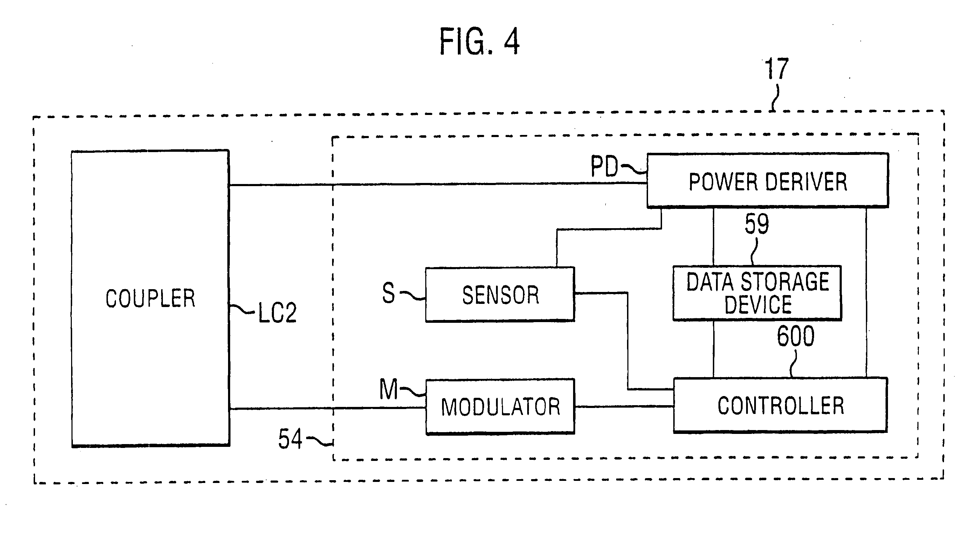

The reader unit 15 comprises a reader 21 which is coupled to the mobile telephone processor 23 via line 23a and which is also coupled to a first coupling element or coupler LC1 arranged to couple to a similar second coupling element LC2 (see Figure 4) carried by the passive data storage device 17 when the replacement fascia or cover portion 3 is fitted to the main body of the mobile telephone.

Although not shown in Figure 2, it will be appreciated that the components of the mobile telephone 1 and the reader unit 15 will be powered by the battery of the mobile telephone 1, although a separate power supply may be provided.

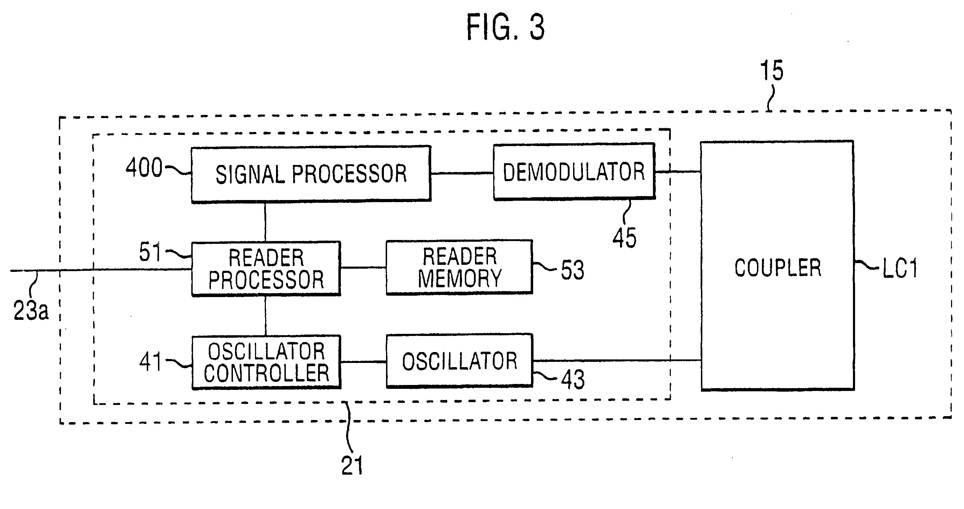

Figure 3 shows an example of the reader unit 15 which consists of the coupling element or coupler LC1 and the reader 21. The reader 21 has a reader microprocessor or microcontroller 51 having a memory 53 which will generally be non-volatile but could be volatile if backed-up, for example battery backed-up. An oscillator 43 controlled by an oscillator controller 41 under the control of the reader microprocessor 51 supplies an oscillator output signal to the coupling element LC1. The reader microprocessor 51 controls the oscillator control 41 to control activation of the oscillator 43 and, in this example, also controls the oscillator control 41 to interrupt the output of the oscillator 43 to provide a signal from which the data storage device 17 within the fascia 3 can derive a clock signal as will be described below.

The first coupling element LC1 is also coupled to a demodulator 45 which enables the modulation to be recovered from an amplitude modulated signal. The recovered modulation is supplied to signal processor 400 which supplies a digital signal representing a string of binary ones and zeros to the reader microprocessor 51. In response to receipt of control data from the passive data storage device, the reader microprocessor 51 communicates with the mobile telephone processor 23 on data line 23a (see Figure 2) so as to affect the functionality or operating characteristics of the mobile telephone.

Figure 4 shows a simplified block diagram of one example of a passive data storage device forming the detectable component 17. As shown, the passive data storage device 17 comprises a passive data storage unit 54 and the coupler or coupling element LC2. The passive data storage unit 54 consists of a power deriver PD that derives a power supply for the passive data storage unit from the oscillator signal coupled from the reader unit 15 (shown in Figure 3) to the passive data storage device 17 by coupling of the first and second coupling element LC1 and LC2, a data store 59 in the form of a non-volatile memory storing control data for supply to the reader unit 15 and a controller 600 for controlling reading of data from the data store 59 and supply of that data to a modulator M that modulates the oscillating signal inductively coupled to the passive data storage device 17. Optionally, the passive data storage device may include a sensor S for reasons that will be set out below.

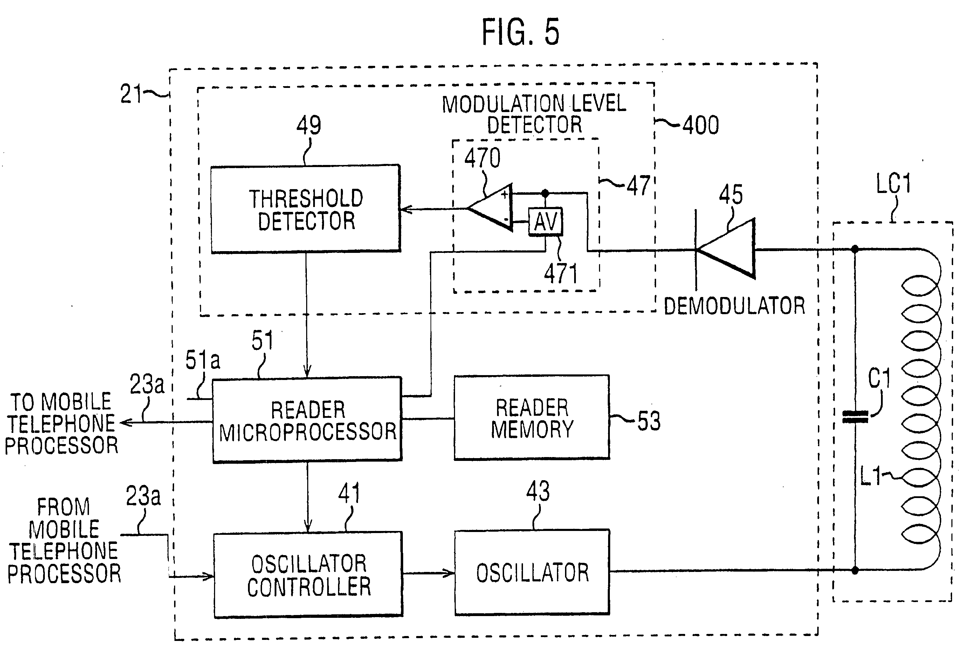

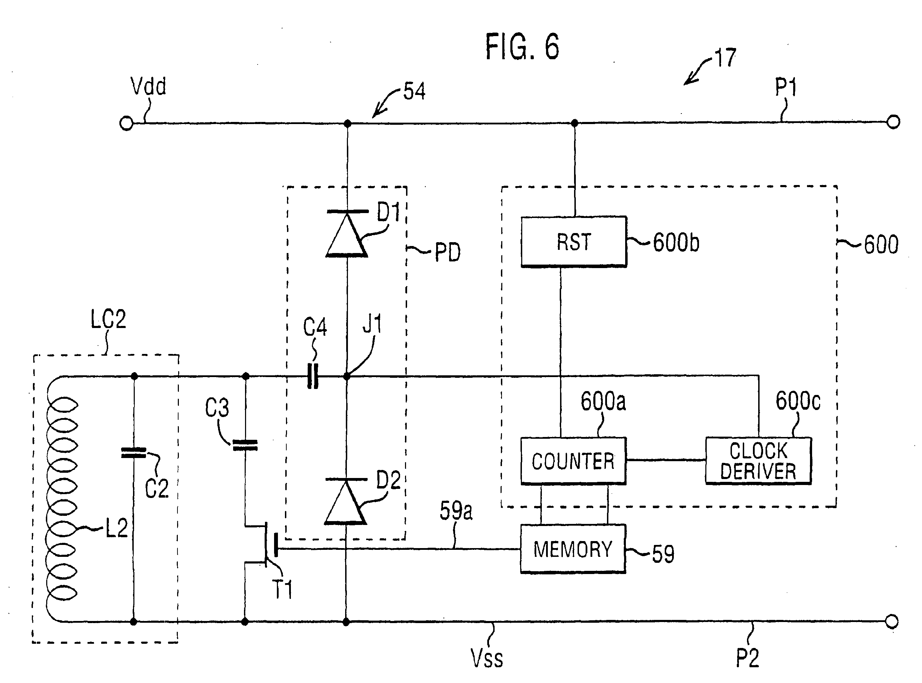

Figures 5 and 6 show, respectively, block diagrams illustrating one example of the reader unit 15 and one example of the passive data storage device 17. In this example, the passive data storage device 17 and reader unit 15 are adapted to communicate via an inductive coupling and each of the couplers LC1 and LC2 consists of a parallel connection of a capacitor and an inductor C1 and L1 and C2 and L2, respectively. The inductive couplers may form a tuned circuit, although this is not necessary at short range. The optional sensor S is not shown in Figure 6.

In the example shown in Figure 5, the demodulator 45 is a simple diode rectifier while the signal processor 400 consists of modulation level and threshold detectors 47 and 49. The modulation level detector 47 comprises a comparator 470 and an averaging circuit 471 controlled by the reader microprocessor 51. The output from the demodulator 45 is supplied to the positive input of the comparator 470. The average of the output of the demodulator 45 is supplied by the averaging circuit 471 to the negative or inverting input of the comparator 470.

The output of the comparator 470 is supplied to the threshold detector 49 which supplies to the reader microprocessor 51 either a one or a zero, depending upon the relationship between the signal and the threshold.

In this example, the oscillator controller 41 is a logic circuit or a transistor switch which controls the oscillator 43 to switch on or off the carrier signal to the first coupling element LC1 in accordance with a signal received from the reader microprocessor 51 and the averaging circuit 471 generally consists of an averaging capacitor connected between the inverted input of the comparator 470 and ground by a transistor switch or transmission gate which is controlled by the reader microprocessor 51 so as to be conducting while the carrier signal is present and after transients have settled but is off while the carrier is off and during carrier turn-on transients so that averaging is only carried out while there is a steady carrier signal.

As shown in Figure 6, the power deriver PD of the passive data storage unit 54 comprises series-connected diodes D1 and D2 and a capacitor C4 coupled between the coupling element LC2 and a junction J1 between the anode of the diode D1 and a cathode of the diode D2. The cathode of the first diode D1 is connected to a first power supply rail P1 (Vdd) while the anode of the second diode D2 is connected to a second power supply rail P2 (Vss). The capacitor C4 and the diodes D1 and D2 act effectively as a voltage doubler enabling the peak to peak voltage of a received AC or oscillating signal inductively coupled to the passive data storage device 17 by the coupling elements LC1 and LC2 to be used by the diodes D1 and D2 to derive a power supply for the passive data storage device 17 from the oscillating signal.

It will, of course, be appreciated that, in the interests of simplicity, the power supply connections to the remaining components of the passive data storage device 17 are not shown in Figure 6.

In this example, the controller 600 comprises a clock signal deriver 600c in the form of a missing pulse detector which is coupled to the junction J1 to derive a clock signal for the data storage unit 54 from the interrupted oscillator signal supplied by the oscillator 43 and an address counter 600a clocked by the clock signal. The controller 600 may also include a reset switch 600b to reset the counter 600a if the passive data storage device 17 is not powered for a predetermined time.

In this example, the controller 600 causes data to be read out from the data store 59 directly to the modulator M which comprises a series-connection of a FET T1 and a capacitor C3 coupled across the capacitor C2 of the coupler LC2. Thus, an output 59a of the data store 59 is coupled to the gate of the FET T1. In this example, the data store 59 is a serial read-only memory (ROM). It may, however, be any form of non-volatile memory that does not require battery backup such as a ROM, an EE-PROM (electrically erasable programmable ROM), a flash memory, F-RAM and so on.

When, as shown in Figure 1, the replaceable fascia 3 is fitted to the main body 5 of the mobile telephone 1 the coupling element LC1 of the passive data storage device 17 lies adjacent and in close proximity to the coupling element LC2 of the reader unit 15, inductively coupling the passive data storage device 17 to the reader unit 15. The oscillator 43 of the reader 21 generates a high or RF (radio frequency), typically 13.56MHz (MegaHertz), signal which is supplied to the first coupling element LC1 and inductively coupled to the passive data storage device 17 via the second coupling element LC2. The voltage doubler formed by capacitor C4 and diodes D1 and D2 thus derives a power supply for the passive data storage device and, when powered, the clock deriver 600c derives a clock signal from the interrupted oscillator signal and control data is output from the data store 59 on output 59a under the control of the counter 600a.

When control data is output by the data store 59 on the output line 59a, the data switches the FET T1. The loading across the inductor L2 varies in dependence upon whether the FET T1 is conducting or non-conducting, causing the oscillating signal to be modulated in accordance with the control data output from the data store 59.

The control data output from the data store 59 is extracted from the modulated oscillating signal by the demodulator 45, modulation level detector 47, threshold detector 49 and reader microprocessor 51 to provide a data input signal to the mobile telephone processor 23 (Figures 2 and 3) representing the data output from the data store 59.

The control data output from the data store 59 and downloaded by the reader microprocessor 51 (Figure 5) to the mobile telephone processor 23 (Figure 2) may then be stored in the writable portion of the memory 29. This control data may comprise at least one of software data, that is computer code executable or implementable by the mobile telephone processor 23, and information data that is stored in the memory 29 so as to be usable by the mobile telephone processor 23. As an example of software data, the data downloaded from the passive data storage device may include upgrades or modifications of the mobile telephone processor software where the fascia is supplied by or under licence from the manufacturer or supplier of the mobile telephone. Additionally or alternatively, software data may include updates or modifications to existing games software provided with the mobile telephone or new games software.

Information data may be provided as an alternative to or in addition to software data. Examples of information data that may be stored by the data store 59 include dialling or ringing tones and telephone numbers, Internet addresses and/or WAP addresses enabling, for example, the supplier of the fascia to attract buyers by supplying new ringing tones and/or to advertise itself or other companies by supplying their telephone numbers, Internet addresses or WAP addresses. Other examples of control data may include graphics data, audio data, image data, video data, biometric data, mobile telephone services, subscription services, network services, promotions, advertisements and so on.

As described above, the control data stored by the passive data storage device consists of the actual software and/or information data or the identity or address of the user's mobile network provider or another third party, service provider. As another possibility, the control data stored by the passive data storage device may be access data that enables the user to access such software and/or information data. For example, this access data may consist of a mobile telephone number or a WAP address that the user of the mobile telephone can contact to download new software data and/or information data or the identity or address of the user's mobile network provider or another, third party, service provider. As another possibility, downloading of access data may cause the mobile telephone processor 23 to make additional facilities for which the mobile telephone processor is already programmed but that are currently barred from the user available to the user, for example facilities such as international calling access, voicemail and message-taking facilities. As a further possibility, such access data may cause the mobile processor 23 to enable the user to access data previously stored in the memory 29 but not accessible to the user. Such data may include information data such as ringing tones, telephone numbers and/or WAP addresses and software data such as modifications to the processing software of the mobile telephone and/or upgrades or modifications to games software already available to the user or new games software.

The access data may be, for example, an identity code that enables the mobile telephone processor 23 to identify the particular type of fascia 3 by comparison with data stored in its memory. The mobile telephone processor 23 may then control the mobile telephone functionality and capability available to the user in accordance with the identified fascia 3.

The mobile telephone processor may be programmed so that, if it receives no control data or receives incorrect or non-understandable control data (for example if it receives the wrong access or identity code data), then the mobile telephone processor 23 may determine that the fascia is a counterfeit or unrecognised fascia and may modify or restrict the functionality of the mobile telephone. The mobile telephone processor 23 may inhibit access to all or certain optional features or functions and may continue to inhibit use of those optional features or functions until a genuine fascia is fitted to the mobile telephone. For example, if the mobile telephone processor 23 determines that the fascia is a counterfeit fascia, then the mobile telephone processor 23 may simply limit the number of functions available via the mobile telephone, for example the mobile telephone processor 23 may limit the available number of ringing or dialling tones or message taking options, for example, or may "lock" the mobile telephone so that only emergency outgoing calls may be made.

As described above, the passive data storage device is powered and so supplies its stored data to the reader unit 15 whenever the first and second coupling elements LC1 and LC2 are inductively coupled. The reader microprocessor may send the received data continually or periodically to the mobile telephone processor 23. Continually powering the passive data storage device 17 may, however, present a drain on the battery of the mobile telephone. Accordingly, the reader microprocessor 51 may be programmed to cause the oscillator control 41 to switch on the oscillator 43 only at predetermined intervals so that the passive data storage device 17 is periodically activated to send its data.

As described above, the passive data storage device 17 is a synchronous passive data storage device, that is the clock signal of the passive data storage device 17 is controlled by and so synchronised with the reader microprocessor 51. However, the passive data storage device 17 may be an asynchronous device, that is the clock deriver 600c coupled to the junction J1 shown in Figure 4 may be replaced by a clock signal generator which is powered up when a power supply is derived by the passive data storage device to generate an independent clock signal for the passive data storage device. Also, although Figure 6 shows the use of a counter 600a to count out data from the data store 59 it will, of course, be appreciated that the data store 59 may be arranged simply to output its data an address at a time and that the counter 600a may not be necessary. In addition, the passive data storage device 17 may incorporate a microcontroller that controls read out of data from the data store 59 which may enable different areas of the data store 59 (and so different data) to be accessed and read out in accordance with instructions supplied by the reader microprocessor 51. Such instructions may be transmitted by representing 0 and 1 as long and short duration interruptions of the oscillator signal.

As described above, the passive data storage device modulates the signal received from the reader by, under control of the counter 600a and clock divider 600c, reading data out of the data store 59.

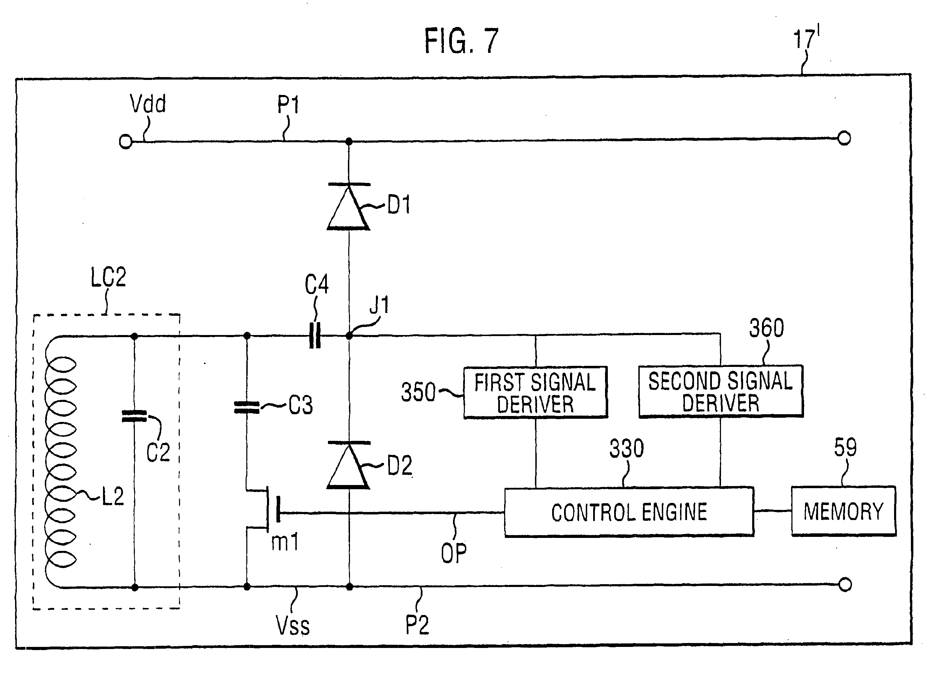

Figure 7 shows an example of another passive data storage device 17' that may be used in place of the passive data storage device 17 shown in Figure 6. This data storage device uses a different controller from that shown in Figure 6. In this case, the controller comprises a control engine 330 arranged to recognise a number of different codes (in the form of predetermined sequences of ones and zeros) transmitted to it by the reader microprocessor 51. In this example, the reader microprocessor 51 is arranged to transmit ones and zeros by causing the oscillator control 41 to interrupt the output of the oscillator 43 for short and long durations, respectively.

In this example, a clock signal for the control engine 330 is derived from the signal supplied by the oscillator 43 by a first signal deriver 350 in the form of a fast missing pulse detector coupled to junction J1 while the control engine 330 is arranged to extract the data transmitted by the reader 17' using the output of the first signal deriver 350 and the output of a second signal deriver 360 in the form of a slow missing pulse detector also coupled to the junction J1.

The timeout periods of the fast and slow missing pulse detectors are set so that the output of the fast missing pulse detector will have one of two widths dependent upon whether the particular data bit is a binary "zero" or a binary "one" while the slow missing pulse detector will provide a pulse only when a particular data bit is a binary "one". The control engine 330 can thus determine from the outputs of the first and second signal derivers 350 and 360 whether a received bit is a binary "zero" or a binary "one". As set out above, the control engine 330 is programmed to enter a number of different states dependent upon the instruction code received from the reader unit 15. These different states may cause the control engine to read out data from different areas of the data store 59. The data store 59 may, however, be an electrically erasable memory in which case at least one of the states of the control engine 330 may enable the control engine to erase the data in a portion of the data store 59 and to write new data supplied by the reader unit 15 into the data store 59. This would enable, for example, the reader unit 15 to overwrite at least a part of the content of the data store 59 after the fascia 3 has first been coupled to the main body 5 so as to limit or inhibit access to the functionality provided by the data stored in the data store 59 if the fascia is subsequently removed and fitted to a different mobile telephone. As another possibility, the mobile telephone processor may be programmed to place a time limit on the availability of the additional functions provided by the data stored in the data store 59.

Although as described above, the control engine 330 is a state machine with its own non-volatile memory it will, of course, be appreciated that the state machine may be replaced by an appropriately programmed microprocessor or microcontroller also having its own memory. Also, different methods for communicating instructions from the reader unit 15 to the control engine 330 may be used.

Where, as described above, the data store 59 is writable, then it may also be used to extend the memory capacity of the mobile telephone to store further telephone numbers and/or other data. The passive storage device 17 may also be used to provide a record of historical degree and frequency of use of the mobile telephone. Further details of the writing and reading operations that may be carried out by the control engine 330 are to be found in UK Patent Application number: 0031518.4 (GB-A-2370462) or the corresponding PCT application number GB01/05690 (WO02/052419. Data may also be written to the mobile telephone SIM card by the reader unit and mobile telephone processor.

As described above, the actual components of the user interface keypads or keyboard are carried by the main body of the mobile telephone and the fascia 3 simply provides apertures through which the keypads extend.

As described above, the reader unit 15 is, as shown in Figure 2, separate from the main components of the mobile telephone. The reader unit 15 may, however, be provided within the main components of the mobile telephone. For example, the reader unit may be provided in the SIM card so that it is not necessary to adapt the reader unit for different hardware.

As described above, the passive data storage device 17 uses amplitude modulation to transmit data to the reader unit 15. It will, however, be appreciated that frequency modulation may be used as may phase modulation as described in WO 97/23060 (PCT/GB96/02975).

As described above, the first and second coupling elements are arranged to couple inductively. The first and second coupling elements may be arranged to couple in any other manner that requires the first and second coupling elements to be in close proximity but not in physical contact with one another. For example, the first and second coupling elements may be arranged to couple capacitively. These non contact arrangements have advantages over ohmic coupling arrangements because the use of ohmic coupling has the disadvantage that actual electrical contact needs to be established between the first and second coupling elements and that this requires the coupling elements to be exposed which may make manufacture of the fascia 3 more difficult and repeated removal and fitting of fascias may cause wear and tear of the coupling elements. Also, both capacitive and ohmic coupling require more accurate alignment and closer positioning of the first and second coupling elements than inductive coupling. Other forms of coupling, such as an optical coupling, may also be used.

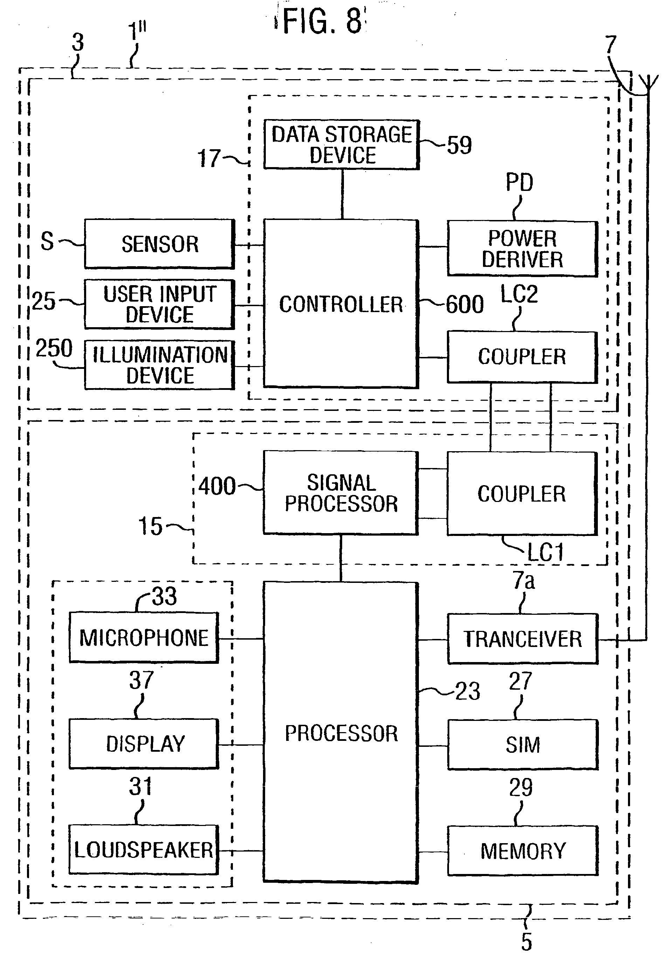

Figure 8 shows a functional block diagram of another example of a mobile telephone 1" not falling within the scope of the claims wherein the oscillator and oscillator control are omitted from the reader unit and the couplers LC1 and LC2 are tuned to the RF signal emitted by the antenna 7 of the mobile telephone so that the components carried by the fascia 3 derive a power supply from the RF signal transmitted by the mobile telephone and data communication between the passive data storage device 17 and the reader unit is achieved in the manner described above with reference to Figures 6 and 7 by varying the loading of the inductive coupler LC2 to provide an amplitude modulated signal.

The examples described may be modified so that the mobile phone processor 23 carries out the functions of the reader processor 51 so that, the reader unit 15 need not include the reader processor 51 and reader memory.

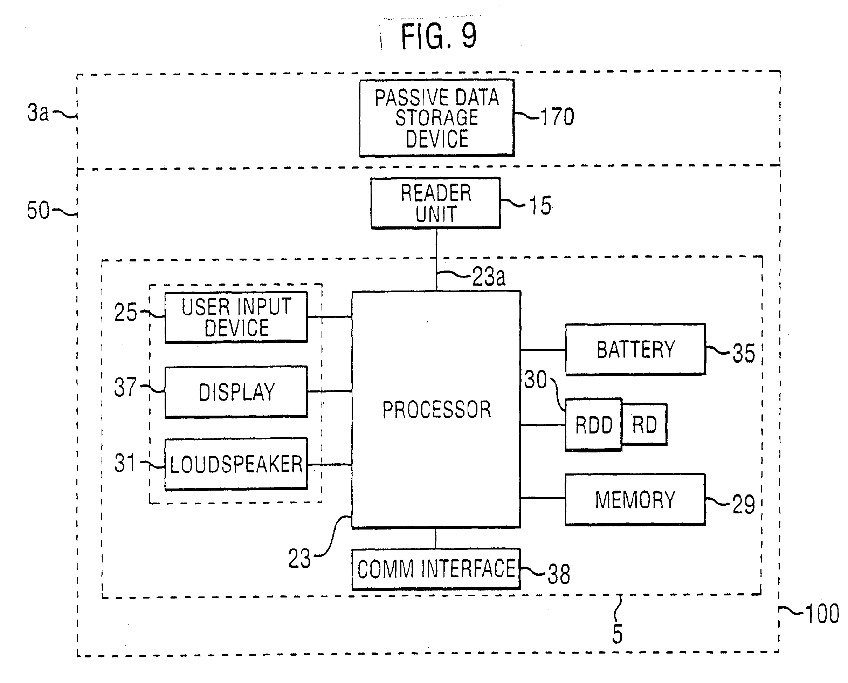

The present invention may be applied to computing devices such as personal computers, PDA (personal digital assistant) or games console with or without facilities for enabling communication over a mobile telecommunications network. Figure 9 shows a functional block diagram of a computing device such as a PDA (personal digital assistant) or games console 100 which differs from the mobile telephone shown in Figure 2 in that the SIM card is replaced by a removable disk drive 30 for receiving a removable disk RD, and the transceiver is replaced by a communications interface 38 which may be a MODEM. In this case, the control data may comprise software data in the form of new software and/or upgrades for existing software provided on the PDA including, for example, games software, wordprocessing, spread sheet and other applications software. The control data may alternatively or additionally include information data such as, for example, tunes and images that may be played via the loudspeaker 31 or shown on the display 37 under control of the processor 23. As described above, the control data may alternatively be in the form of access data that enables the reader unit 15 to instruct the processor 23 where to obtain data represented by the access data by, for example, accessing a particular site on the Internet or another network using the communications interface. Such data may be pre-stored in the memory 29, or available via the communications interface 38. The reader unit and data storage device of this computing apparatus may have any of the configurations described above with the mobile telecommunications transceiver being replaced by the communications interface.

Examples, not falling within the claims, will now be described of electronic devices or electrical appliances having an attachable functional component such as, for example, a replaceable tool will be described.

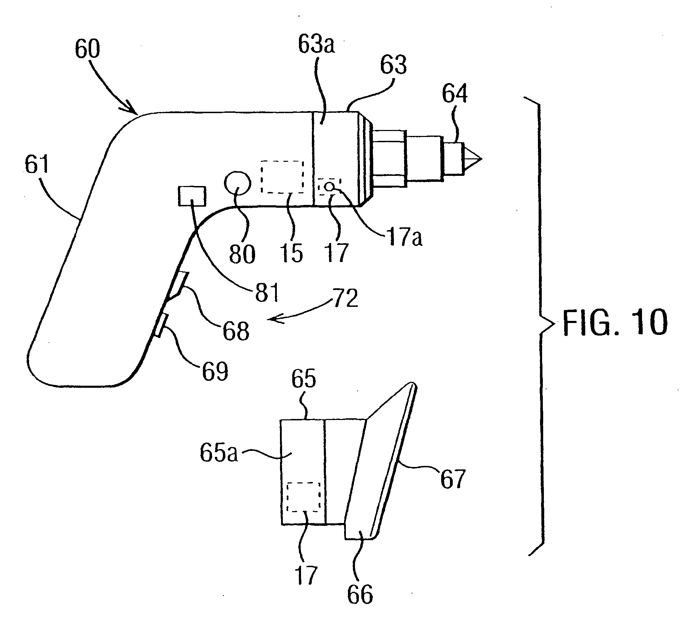

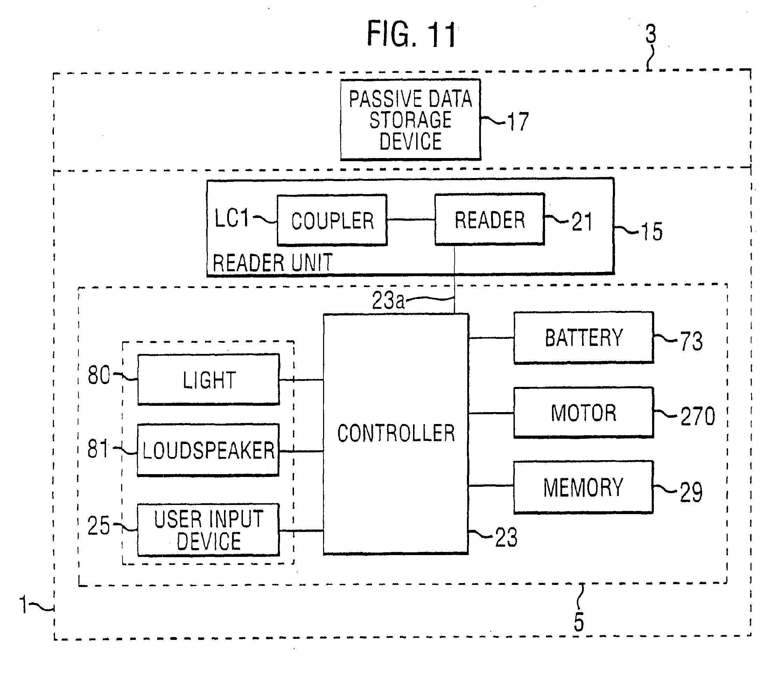

Figure 10 shows a diagrammatic perspective view of a domestic power tool 60 while Figure 11 shows a functional block diagram of the electrical appliance 60. The electrical appliance may be mains or battery powered, having a number of different attachable components comprising or arranged to carry tools for performing different tasks. As shown in Figure 10, the power tool main body 61 is carrying a replaceable component in the form of a chuck assembly 63 having a chuck 64 for receiving, for example, drill bits or screwdriver heads. Figure 10 also shows a further attachable component that may be fitted to the main body 61 of the power tool in place of the chuck assembly. In the example shown, the further attachable component 65 is a sander assembly having a sander foot 66 for receiving a sanding sheet 67.

In the case of this power tool, the main body 61 has an outer plastics, generally moulded, casing. Coupling between the main body 61 and an attachable component 63 or 65 is effected via the drive shaft of a motor (not shown in Figure 10) of the power tool. When an attachable component 63 or 65 is fitted to the main body, a plastics coupling collar 63a or 65a of the replaceable component is fitted onto a portion of the casing of the main body 61. A passive data storage device 17 is fitted to, generally embedded within, the coupling collar 63a or 65a while a reader unit 15 is housed within the casing of the main body together with the other control circuitry of the power tool.

The reader unit 15 is positioned such that, when the attachable component 63 or 65 is fitted to the main body 61, the passive data storage device 17 will be in range of, as shown will lie adjacent to, the reader unit 15 so that, as will be described in detail below, couplers of the passive data storage device 17 and reader unit 15 couple to enable the passive data storage device 17 to derive a power supply from a signal supplied by the reader unit 15 and, when so activated, to transmit control data contained in its memory.

As can be seen from Figures 10 and 11, the main body 61 comprises, in addition to the reader unit 15, a controller such as a microprocessor or microcontroller 23 which controls the overall operation of the power tool 60. The controller 23 may be associated with a non-volatile memory 29 which has a read-only portion which contains control operation software data and information data and a writable portion. The controller 23 is coupled to a motor 270 for driving the attachable tool in known manner and also to a user interface 25 consisting of a user input device 72 which will generally, as is known in the art and as shown in Figure 10, consist of an on/off switch 68 and possibly also a speed control 69. The user interface may also include one or more indicator lights 80 and a rudimentary loudspeaker 81 that, under control of the controller 23, may issue audible warnings to the user under certain circumstances. In this example, the power tool 60 is battery operated and the control circuitry 50 in the main body also includes a battery 73. In the interest of simplicity, the couplings between the battery 73 and the various components of the control circuitry, apart from the controller 23, are not shown in Figure 11.

As described earlier, the reader unit 15 comprises a reader 21 which is coupled to the controller 23 via line 23a and to a first coupler or coupling element LC1. The first coupling element LC1 is arranged to couple to a similar second coupling element LC2 (see Figure 4) carried by the passive data storage device 17 when the attachable tool 63 or 65 is fitted to the main body of the appliance. In this embodiment, the reader unit 15 and the passive data storage device 17 are as described above with reference to Figures 3 to 6.

When the attachable component is fitted to the main body of the electrical appliance the coupling element LC1 of the passive data storage device 17 lies adjacent and in close proximity to the coupling element LC2 of the reader unit 15, inductively coupling the passive data storage device 17 to the reader unit 15 and data is output by the passive data storage device 17 and read by the reader unit as described above with reference to Figures 3 to 6.

The control data output from the data store 59 is extracted from the modulated oscillating signal by the demodulator 45, modulation level detector 47, threshold detector 49 and reader microprocessor 51 to provide a data input signal to the controller 23 representing the data output from the data store 59.

The control data output from the data store 59 and downloaded by the reader microprocessor 51 to the controller 23 may then be stored in the writable portion of the memory 29. This control data may comprise at least one of software data, that is computer code executable or implementable by the controller 23, and information data that is stored in the memory 29 so as to be usable by the controller 23. As an example of software data, the data downloaded from the passive data storage device may include upgrades or modifications of any electrical appliance software where the attachable component is supplied by or under licence from the manufacturer or supplier of the electrical appliance.

The control data may be, for example, an identity code that enables the controller 23 to identify the particular type of attachable tool by comparison with data stored in its memory. The controller 23 may then control the functionality and capability available to the user in accordance with the attachable tool.

As an example, the controller 23 may control at least one of the speed, drive direction and duration of the operation of the motor 270 in accordance with the control data received by the reader microprocessor. Alternatively or additionally, the controller 23 may inhibit operation of the electrical appliance if the reader microprocessor receives no control data or receives incorrect or non-understandable control data, for example if the reader microprocessor receives the wrong access or identity code data. This would ensure that, for safety reasons, the electrical appliance is not accidentally operated with an inappropriate tool fitted and for both safety and power saving purposes would ensure that the electrical appliance is not accidentally operated when no tool is attached. Where an incorrect tool is fitted, then the reader microprocessor may cause the controller 23 to cause the warning light 80 to flash or the loudspeaker 81 to emit an audio signal such as an audible warning beep.

Where, as described above, the data store 59 is writable, then the passive storage device may also be used to provide a record of historical degree and frequency of use of the attachable tool. Further details of the writing and reading operations that may be carried out by the control engine 330 are to be found in UK Patent Application number: 0031518.4 (GB-A-2370462) and the corresponding PCT application Number GB01/05690 (WO02/052419).

The electronic devices or electrical appliances may be mobile communications devices and other electrical appliances and electronic devices having a microprocessor or microcontroller where a portion or the whole of the housing or a cover of the device is replaceable or where an attachable component such as a tool or a plug-in key or component can be attached to or fitted into or onto the device.

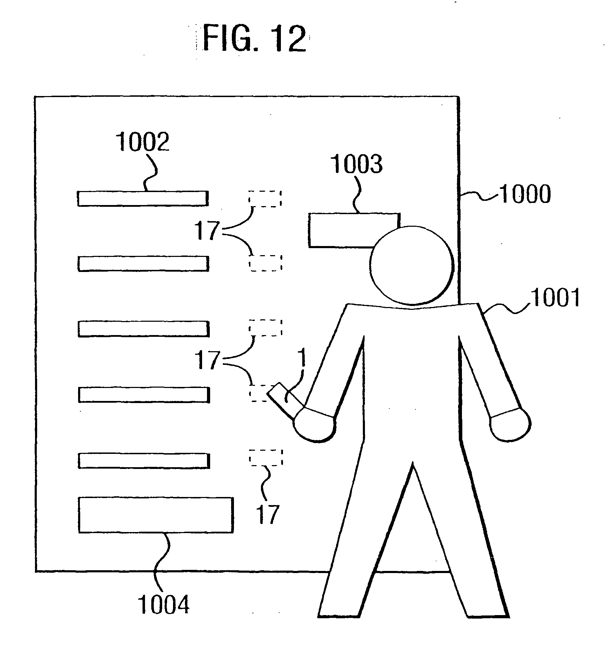

In embodiments of the present invention, the electronic device has communications facilities and the data read by the reader unit may be used to communicate with another device, for example to enable payment for a service or product to be effected. Thus, in one embodiment of the invention as shown very schematically in Figure 12, a unit in the form of a product or ticket vending machine 1000 may carry one or more passive data storage devices 17 each protected by an outer skin or coating of the vending machine housing that enables transmission of a radio frequency signal and so shown in dashed lines in Figure 12. Each passive data storage device 17 is associated with a corresponding box 1002 containing a brief description of the corresponding ticket or product. As shown the passive data storage devices are to the side of the boxes. They may however be within the boxes. When a user 1001 wishes to purchase a product or ticket from the vending machine he brings his portable communications device 1 into close proximity with the data storage device 17 associated with the desired product or ticket to enable the data storage device to derive a power supply and transmit data to the reader unit in the portable communications device. The reader unit in the portable communications device supplies the read data to the mobile telephone processor which accesses a location on the communications network identified by the data, for example a service provider which may then debit the user's account and return an authorisation code that when entered by the user using a key pad 1003 causes the vending machine to supply the corresponding required product or ticket to an outlet 1004.

Instead of being a vending machine, the unit 1000 may be an advertising hoarding or the like with the passive data storage devices embedded in a cardboard or paper poster. In this case, the keypad and outlet will of course not be present and the user will be coupled by the mobile telephone transceiver to source of information for example a WAP or website corresponding to a selected passive data storage device. In this case, the passive data storage device may be associated with images of different products or services, for example.

Mobile communication devices having reader units as described above may also communicate with one another. Communication by RF or similar electromagnetic frequency has advantages over more sophisticated technology such as BlueTooth^{™} technology in that the RF communication system is cheaper and is shorter range so that there is less possibility of interference or crosstalk with other devices. Typically a reader unit will be able to communicate with a data storage device or other reader unit over a range of up to about six inches. Also, in contrast to BlueTooth^{™} which uses a frequency of about 2.4 GigaHertz, uses frequency hopping and a large number, typically 82, channels, RF communication uses a fixed single frequency, typically 13.56 MegaHertz, which provides a continuous signal which is modulated, for example amplitude modulated, by the data to be transmitted. In addition, unlike BlueTooth^{™}, such communications do not require the use of an external protocol. All that is required is that the reader unit can demodulate the modulated carrier signal supplied by the data storage device.

Such a mobile communications device incorporating a reader unit may be used in many forms of commerce, as a payment method, to obtain account details and so on.

## Claims

1. A vending system comprising:
a vending device for enabling at least one product or at least one item relating to a product or service to be supplied to a user, the vending device carrying passive data storage means having a memory storing data relating to the at least one product or service; and a portable communications device having communication means for communicating over a network and signal supplying means for supplying a carrier signal, the passive data storage means carried by the vending device having power deriving means for deriving a power supply for the passive data storage means from the carrier signal when the portable communications device is in the vicinity of the passive data storage means and modulation means for modulating the carrier signal in accordance with data read from the memory in response to derivation of a power supply by the power supply deriving means, the portable communications device having data extracting means for extracting data from a modulated carrier signal and control means operable to enable the user to be supplied with the at least one product or the at least one item relating to a product or service.

2. A vending system according to claim 1, wherein the control means is operable to cause the communications means to access a location on the network identified by data extracted by the data extracting means to enable the user to be supplied with the at least one product or the at least one item relating to a product or service.

3. A vending system according to claim 1 or 2, wherein the vending device has a user input for enabling a user to input authorisation data to enable the vending of the at least one product or the at least one item relating to a product or service.

4. A vending system according to claim 1 or 2, wherein the vending device has a user input for enabling a user to input authorisation data received by the communications means to enable the vending of the product or the at least one item relating to a product or service.

5. A vending system according to claim 2, 3 or 4, wherein the location on the network is associated with a service provider and wherein the service provider is operable to debit an account in response to the communications means accessing the location on the network.

6. A vending system according to any of the preceding claims, wherein the vending device has a vending outlet for supplying the at least one product or the at least one item relating to a product or service directly to the user.

7. A vending system according to any of the preceding claims, wherein the vending device carries plural passive data storage devices each associated with a different product or service.

8. A vending system according to any of the preceding claims, wherein the vending device has a user interface presenting to the user information relating to the or each product or service.

9. A vending system according to any of claims 1 to 6, wherein the vending device carries plural passive data storage devices each associated with a different product or service and the vending device has a respective description of the corresponding product or service associated with each passive data storage device.

10. A vending system according to any of the preceding claims, wherein the portable communications device comprises at least one of a mobile telephone and a personal digital assistant.

11. A vending system according to any of the preceding claims, wherein the passive data storage means and the portable communications device are operable to couple inductively when the portable communications device is in the vicinity of the passive data storage means.

12. A vending device for use in a vending system according to claim 1 for enabling at least one product or at least one item relating to a product or service to be supplied to a user, the vending device carrying passive data storage means having a memory storing data relating to the at least one product or service, the passive data storage means carried by the vending device having power deriving means for deriving a power supply for the passive data storage means from a carrier signal supplied by signal supplying means of a portable communications device when the portable communications device is in the vicinity of the passive data storage means and modulation means for modulating the carrier signal in accordance with data read from the memory in response to derivation of a power supply by the power supply deriving means so as to enable the read data to be communicated to the portable communications device, the vending device having vending means operable to enable the user to be supplied with the at least one product or the at least one item relating to a product or service.

13. A vending device according to claim 12, wherein the supply of the at least one product or the at least one item relating to a product or service is in response to authorisation data received by a network communication means of the portable communications device from a location on the network identified by data read from the memory.

14. A vending device according to claim 12 or 13, wherein the vending means comprises a user input for enabling a user to input authorisation data received by the communication means to enable the vending of the at least one product or service.

15. A vending system according to claim 12, 13 or 14, wherein the vending means has a vending outlet for supplying the at least one product or the at least one item relating to a product or service to the user in response to authorisation data received by the communication means.

16. A portable communications device for use in a vending system according to claim 1 comprising a vending device carrying passive data storage means having a memory storing data relating to the at least one product or service, the portable communications device having communication means for communicating over a network and signal supplying means for supplying a carrier signal which, when the portable communications device is in the vicinity of the passive data storage means, causes power deriving means of the passive data storage means to derive a power supply from the carrier signal and to modulate the carrier signal in accordance with data read from the memory, the portable communications device also having data extracting means for extracting data from a modulated carrier to enable the user to be supplied with the at least one product or service.

17. A portable communications device for use in a vending system according to claim 16, wherein the portable communications device comprises control means for causing the communication means to access a location on the network identified by the data extracted by the data extracting means to enable the user to be supplied with the at least one product or the at least one item relating to a product or service in response to authorisation data received by the communication means.

18. A payment system comprising a portable communications device having communication means for communicating over a network and signal supplying means for supplying a carrier signal which, when the portable communications device is in the vicinity of passive data storage means, causes power deriving means of the passive data storage means to derive a power supply from the carrier signal and the passive data storage means to modulate the carrier signal in accordance with data read from a memory of the passive data storage means, the portable communications device also having data extracting means for extracting data from a modulated carrier signal and control means operable to use data extracted by the data extracting means to enable payment for at least one product or service to be effected by the portable communications device.

19. A payment system according to claim 18, wherein the control means operable to use data extracted by the data extracting means to enable the communications means to access a location on the network to enable payment to be effected.

## Patentansprüche

1. Verkaufssystem, das umfasst:
ein Verkaufsgerät, das ermöglicht, dass mindestens ein Produkt oder mindestens ein Gegenstand, der ein Produkt oder eine Dienstleistung betrifft, an einen Benutzer geliefert wird, wobei das Verkaufsgerät ein passives Datenspeichermittel mit einem Daten speichernden Speicher trägt, der das mindestens eine Produkt oder die mindestens eine Dienstleistung betrifft; und ein tragbares Kommunikationsgerät mit einem Kommunikationsmittel zum Kommunizieren über ein Netzwerk und ein Signalliefermittel zum Liefern eines Trägersignals, wobei das passive Datenspeichermittel, das von dem Verkaufsgerät getragen wird, ein Energieableitungsmittel zum Ableiten einer Energieversorgung für das passive Datenspeichermittel vom Trägersignal aufweist, wenn sich das tragbare Kommunikationsgerät in der Nähe des passiven Datenspeichermittels und des Modulationsmittel zum Modulieren des Trägersignals in Übereinstimmung mit Daten befindet, die als Antwort auf eine Ableitung einer Energieversorgung durch das Energieversorgungsableitungsmittel aus dem Speicher gelesen werden, wobei das tragbare Kommunikationsgerät ein Datenextraktionsmittel zum Extrahieren von Daten aus einem modulierten Trägersignal und ein Steuermittel aufweist, das betriebsbereit ist, um zu ermöglichen, dass der Benutzer mit dem mindestens einen Produkt oder dem mindestens einen Gegenstand, der ein Produkt oder eine Dienstleistung betrifft, beliefert wird.

2. Verkaufssystem nach Anspruch 1, wobei das Steuermittel betriebsbereit ist, um zu bewirken, dass das Kommunikationsmittel Zugriff auf einen Ort im Netzwerk hat, der von den Daten identifiziert wird, die vom Datenextraktionsmittel extrahiert wurden, um zu ermöglichen, dass der Benutzer mit dem mindestens einem Produkt oder dem mindestens einem Gegenstand, der ein Produkt oder eine Dienstleistung betrifft, beliefert wird.

3. Verkaufssystem nach Anspruch 1 oder 2, wobei das Verkaufsgerät eine Benutzereingabe aufweist, die es einem Benutzer ermöglicht, Autorisationsdaten einzugeben, um das Verkaufen von dem mindestens einen Produkt oder dem mindestens einen Gegenstand, der ein Produkt oder eine Dienstleistung betrifft, einzugeben.

4. Verkaufssystem nach Anspruch 1 oder 2, wobei das Verkaufsgerät eine Benutzereingabe aufweist, die ermöglicht, dass ein Benutzer Autorisationsdaten eingibt, die von dem Kommunikationsmittel erhalten werden, um das Verkaufen des Produkts oder des mindestens einen Gegenstands, der ein Produkt oder eine Dienstleistung betrifft, zu ermöglichen.

5. Verkaufssystem nach Anspruch 2, 3 oder 4, wobei der Ort im Netzwerk in Beziehung mit einem Dienstleistungsanbieter steht und wobei der Dienstleistungsanbieter betriebsbereit ist, um als Antwort auf das Kommunikationsmittel, das auf den Ort im Netzwerk zugreift, ein Konto zu belasten.

6. Verkaufssystem nach einem der vorhergehende Ansprüche, wobei das Verkaufsgerät einen Verkaufsauslass zum Liefern des mindestens einen Produkts oder des mindestens einen Gegenstands, der ein Produkt oder eine Dienstleistung betrifft, direkt an den Benutzer.

7. Verkaufssystem nach einem der vorhergehenden Ansprüche, wobei das Verkaufsgerät mehrfache passive Datenspeichergeräte trägt, wobei jedes einem anderen Produkt oder einer anderen Dienstleistung zugeordnet ist.

8. Verkaufssystem nach einem der vorhergehenden Ansprüche, wobei das Verkaufsgerät eine Benutzerschnittstelle aufweist, die die Benutzerinformation, die das oder jedes Produkt oder die oder jede Dienstleistung betrifft, darstellt.

9. Verkaufssystem nach einem der Ansprüche 1 bis 6, wobei das Verkaufsgerät mehrfache passive Datenspeichergeräte trägt, wobei jedes einem anderen Produkt oder einer anderen Dienstleistung zugeordnet ist, und das Verkaufsgerät eine zugehörige Beschreibung des entsprechenden Produkts oder der entsprechenden Dienstleistung aufweist, die jedem passiven Datenspeichergerät zugeordnet ist.

10. Verkaufssystem nach einem der vorhergehenden Ansprüche, wobei das tragbare Kommunikationsgerät mindestens eines von beiden, ein Mobiltelefon oder einen Personal Digital Assistant, umfasst.

11. Verkaufssystem nach einem der vorhergehenden Ansprüche, wobei das passive Datenspeichergerät und das tragbare Kommunikationsgerät betriebsbereit sind, um sich induktiv zu koppeln, wenn sich das tragbare Kommunikationsgerät in der Nähe des passiven Datenspeichermittels befindet.

12. Verkaufsgerät zur Verwendung in einem Verkaufssystem nach Anspruch 1, das ermöglicht, dass mindestens ein Produkt oder mindestens ein Gegenstand, der ein Produkt oder eine Dienstleistung betrifft, an einen Benutzer geliefert wird, wobei das Verkaufsgerät ein passives Datenspeichermittel trägt, das einen Speicher aufweist, der Daten speichert, die das mindestens eine Produkt oder die mindestens eine Dienstleistung betreffen, wobei das passive Datenspeichermittel, das von dem Verkaufsgerät getragen wird, ein Energieableitungsmittel zum Ableiten einer Energieversorgung für das passive Datenspeichermittel von einem Trägersignal, das von einem Signalliefermittel eines tragbaren Kommunikationsmittel geliefert wird, aufweist, wenn sich das tragbare Kommunikationsgerät in der Nähe des passiven Datenspeichermittels und des Modulationsmittels zum Modulieren des Trägersignals in Übereinstimmung mit Daten, die als Antwort auf die Ableitung einer Energieversorgung durch das Energieversorgungsableitungsmittel aus dem Speicher gelesen werden, befindet, um zu ermöglichen, dass die gelesenen Daten zum tragbaren Kommunikationsgerät kommuniziert werden, wobei das Verkaufsgerät Verkaufsmittel aufweist, die betriebsbereit sind, um zu ermöglichen, dass der Benutzer mit dem mindestens einen Produkt oder dem mindestens einen Gegenstand, der ein Produkt oder eine Dienstleistung betrifft, beliefert wird.

13. Verkaufsgerät nach Anspruch 12, wobei das Liefern des mindestens einen Produkts oder des mindestens einen Gegenstands, der ein Produkt oder eine Dienstleistung betrifft, als Antwort auf die Autorisationsdaten eintritt, die von einem Netzwerkkommunikationsmittel des tragbaren Kommunikationsgeräts von einem Ort im Netzwerk erhalten werden, der von den Daten, die aus dem Speicher gelesen wurden, identifiziert wurden.

14. Verkaufsgerät nach Anspruch 12 oder 13, wobei das Verkaufsmittel eine Benutzereingabe umfasst, die ermöglicht, dass ein Benutzer Autorisationsdaten, die von dem Kommunikationsmittel erhalten werden, einzugeben, um das Verkaufen des mindestens einen Produkts oder der mindestens einen Dienstleistung zu ermöglichen.

15. Verkaufssystem nach Anspruch 12, 13 oder 14, wobei das Verkaufsmittel einen Verkaufsauslass zum Liefern des mindestens einen Produkts oder des mindestens einen Gegenstands, der ein Produkt oder eine Dienstleistung betrifft, an den Benutzer als Antwort auf die Autorisationsdaten, die von dem Kommunikationsmittel empfangen wurden, aufweist

16. Tragbares Kommunikationsgerät zur Verwendung in einem Verkaufssystem nach Anspruch 1, das ein Verkaufsgerät umfasst, das ein passives Datenspeichermittel trägt, das einen Speicher aufweist, der Daten speichert, die das mindestens eine Produkt oder die mindestens eine Dienstleistung betrifft, wobei das tragbare Kommunikationsgerät ein Kommunikationsmittel zum Kommunizieren über ein Netzwerk und ein Signalliefermittel zum Liefern eines Trägersignals aufweist, das, wenn sich das tragbare Kommunikationsgerät in der Nähe des passiven Datenspeichermittels befindet, bewirkt, dass ein Energieableitungsmittel des passiven Datenspeichermittels eine Energieversorgung von dem Trägersignal ableitet und das Trägersignal im Übereinstimmung mit Daten, die aus dem Speicher gelesen werden, moduliert, wobei das tragbare Kommunikationsgerät auch ein Datenextraktionsrmittel aufweist, um Daten von einem modulierten Träger zu extrahieren, um zu ermöglichen, dass der Benutzer mit dem mindestens einen Produkt oder der mindestens einen Dienstleistung beliefert wird.

17. Tragbares Kommunikationsgerät zur Verwendung in einem Verkaufssystem nach Anspruch 16, wobei das tragbare Kommunikationsgerät Steuermittel umfasst, die bewirken, dass das Kommunikationsmittel auf einen Ort im Netzwerk zugreift, der von den Daten identifiziert wird, die vom Datenextraktionsmittel extrahiert werden, um zu ermöglichen, dass der Benutzer mit dem mindestens einen Produkt oder dem mindestens einen Gegenstand, der ein Produkt oder eine Dienstleistung betrifft, als Antwort auf Autorisationsdaten, die von dem Kommunikationsmittel erhalten werden, beliefert wird.

18. Zahlungssystem, das ein tragbares Kommunikationssystem umfasst, das ein Kommunikationsmittel zum Kommunizieren über ein Netzwerk und Signalliefermittel zum Liefern eines Trägersignals aufweist, das, wenn sich das tragbare Kommunikationsgerät in der Nähe des passiven Datenspeichermittels befindet, bewirkt, dass ein Energieableitungsmittel des passiven Datenspeichermittels eine Energieversorgung von dem Trägersignal und dem passiven Datenspeichermittel ableitet, um das Trägersignal in Übereinstimmung mit Daten, die aus einem Speicher des passiven Datenspeichermittels gelesen werden, zu modulieren, wobei das tragbare Kommunikationssystem auch ein Datenextraktionsmittel zum Extrahieren von Daten aus einem modulierten Trägersignal und ein Steuermittel aufweist, das betriebsbereit ist, um Daten zu verwenden, die von dem Datenextraktionsmittel extrahiert wurden, um zu ermöglichen, dass eine Zahlung für das mindestens eine Produkt oder die mindestens eine Dienstleistung durch das tragbare Kommunikationsgerät bewirkt wird.

19. Zahlungssystem nach Anspruch 18, wobei das Steuermittel betriebsbereit ist, um Daten zu verwenden, die von dem Datenextraktionsmittel extrahiert wurden, um zu ermöglichen, dass das Kommunikationsmittel auf einen Ort im Netzwerk zugreift, um zu ermöglichen, dass eine Zahlung bewirkt wird.

## Revendications

1. Système de vente comportant:
un dispositif de vente pour permettre à au moins un produit ou au moins un article concernant un produit ou un service d'être fourni à un utilisateur, le dispositif de vente portant un moyen de stockage passif de données ayant une mémoire stockant des données concernant le, au moins un, produit ou service; et un dispositif de communications portable ayant un moyen de communication pour communiquer par un réseau et un moyen de fourniture de signal pour fournir un signal porteur, le moyen de stockage passif de données porté par le dispositif de vente ayant un moyen de dérivation de puissance pour dériver une alimentation en énergie pour le moyen de stockage passif de données à partir du signal porteur lorsque le dispositif de communications portable se trouve au voisinage du moyen de stockage passif de données et un moyen de modulation pour moduler le signal porteur conformément à des données extraites de la mémoire en réponse à une dérivation d'une alimentation en énergie par le moyen de dérivation d'alimentation en énergie, le dispositif de communications portable ayant un moyen d'extraction de données destiné à extraire des données d'un signal porteur modulé et un moyen de commande pouvant être mis en oeuvre pour permettre à l'utilisateur de recevoir le, au moins un, produit ou le, au moins un, article concernant un produit ou un service.

2. Système de vente selon la revendication 1, dans lequel le moyen de commande peut être mis en oeuvre pour amener le moyen de communication à accéder à un emplacement sur le réseau identifié par des données extraites par le moyen d'extraction de données afin de permettre à l'utilisateur de recevoir le, au moins un, produit ou le, au moins un, article concernant un produit ou un service.

3. Système de vente selon la revendication 1 ou 2, dans lequel le dispositif de vente comporte une entrée d'utilisateur pour permettre à un utilisateur l'entrée de données d'autorisation pour permettre la vente du, au moins un, produit ou du, au moins un, article concernant un produit ou un service.

4. Système de vente selon la revendication 1 ou 2, dans lequel le dispositif de vente comporte une entrée d'utilisateur pour permettre à un utilisateur l'entrée de données d'autorisation reçues par le moyen de communication afin de valider la vente du produit ou du, au moins un, article concernant un produit ou un service.

5. Système de vente selon la revendication 2, 3 ou 4, dans lequel l'emplacement sur le réseau est associé à un fournisseur de service et dans lequel le fournisseur de service peut être mis en oeuvre pour débiter un compte en réponse à l'accès par le moyen de communication à l'emplacement sur le réseau.

6. Système de vente selon l'une quelconque des revendications précédentes, dans lequel le dispositif de vente comporte une sortie de vente pour la fourniture du, au moins un, produit ou du, au moins un, article concernant un produit ou un service, directement à l'utilisateur.

7. Système de vente selon l'une quelconque des revendications précédentes, dans lequel le dispositif de vente porte plusieurs dispositifs de stockage passif de données associés chacun à un produit ou service différent.

8. Système de vente selon l'une quelconque des revendications précédentes, dans lequel le dispositif de vente comporte une interface d'utilisateur présentant à l'utilisateur une information concernant le ou chaque produit ou service.

9. Système de vente selon l'une quelconque des revendications 1 à 6, dans lequel le dispositif de vente porte plusieurs dispositifs de stockage passif de données associés chacun à un produit ou service différent et le dispositif de vente comporte une description respective du produit ou service correspondant associé à chaque dispositif de stockage passif de données.

10. Système de vente selon l'une quelconque des revendications précédentes, dans lequel le dispositif de communications portable comprend au moins l'un d'un téléphone mobile et d'un assistant numérique personnel.

11. Système de vente selon l'une quelconque des revendications précédentes, dans lequel le moyen de stockage passif de données et le dispositif de communications portable peuvent être mis en oeuvre pour être couplés par induction lorsque le dispositif de communications portable se trouve au voisinage du moyen de stockage passif de données.

12. Dispositif de vente destiné à être utilisé dans un système de vente selon la revendication 1, pour permettre à au moins un produit ou au moins un article concernant un produit ou un service d'être fourni à un utilisateur, le dispositif de vente portant un moyen de stockage passif de données ayant une mémoire stockant des données concernant le, au moins un, produit ou service, le moyen de stockage passif de données porté par le dispositif de vente ayant un moyen de dérivation d'énergie pour dériver une alimentation en énergie pour le moyen de stockage passif de données à partir d'un signal porteur fourni par un moyen de fourniture de signal d'un dispositif de communications portable lorsque le dispositif de communications portable se trouve au voisinage du moyen de stockage passif de données et un moyen de modulation destiné à moduler le signal porteur en fonction de données extraites de la mémoire en réponse à une dérivation d'une alimentation en énergie par le moyen de dérivation d'alimentation en énergie afin de permettre aux données lues d'être communiquées au dispositif de communications portable, le dispositif de vente ayant un moyen de vente pouvant être mis en oeuvre pour permettre à l'utilisateur de recevoir le, au moins un, produit ou le, au moins un, article concernant un produit ou un service.

13. Système de vente selon la revendication 12, dans lequel la fourniture du, au moins un, produit ou du, au moins un, article concernant un produit ou un service a lieu en réponse à des données d'autorisation reçues par un moyen de communication par un réseau du dispositif de communications portable depuis un emplacement sur le réseau identifié par des données extraites de la mémoire.

14. Système de vente selon la revendication 12 ou 13, dans lequel le moyen de vente comporte une entrée d'utilisateur pour permettre à un utilisateur l'entrée de données d'autorisation reçues par le moyen de communication afin de valider la vente du, au moins un, produit ou service.

15. Système de vente selon la revendication 12, 13 ou 14, dans lequel le moyen de vente comporte une sortie de vente pour la fourniture du, au moins un, produit ou du, au moins un, article concernant un produit ou un service à l'utilisateur en réponse à des données d'autorisation reçues par le moyen de communication.

16. Dispositif de communications portable destiné à être utilisé dans un système de vente selon la revendication 1, comportant un dispositif de vente portant un moyen de stockage passif de données ayant une mémoire stockant des données concernant le, au moins un, produit ou service, le dispositif de communications portable ayant un moyen de communication pour communiquer par un réseau et un moyen de fourniture de signal destiné à fournir un signal porteur qui, lorsque le dispositif de communications portable se trouve au voisinage du moyen de stockage passif de données, amène un moyen de dérivation d'énergie du moyen de stockage passif de données à dériver une alimentation en énergie à partir du signal porteur et à moduler le signal porteur conformément à des données extraites de la mémoire, le dispositif de communications portable ayant également un moyen d'extraction de données destiné à extraire des données d'une porteuse modulée pour permettre à l'utilisateur de recevoir le, au moins un, produit ou service.

17. Dispositif de communications portable destiné à être utilisé dans un système de vente selon la revendication 16, dans lequel le dispositif de communications portable comporte un moyen de commande destiné à amener le moyen de communication à accéder à un emplacement sur le réseau identifié par les données extraites par le moyen d'extraction de données afin de permettre à l'utilisateur de recevoir le, au moins un, produit ou le, au moins un, article concernant un produit ou un service en réponse à des données d'autorisation reçues par le moyen de communication.

18. Système de paiement comportant un dispositif de communications portable ayant un moyen de communication pour une communication par un réseau et un moyen de fourniture de signal destiné à fournir un signal porteur qui, lorsque le dispositif de communications portable se trouve au voisinage du moyen de stockage passif de données, amène un moyen de dérivation d'énergie du moyen de stockage passif de données à dériver une alimentation en énergie à partir du signal porteur et le moyen de stockage passif de données à moduler le signal porteur conformément à des données extraites d'une mémoire du moyen de stockage passif de données, le dispositif de communications portable ayant aussi un moyen d'extraction de données destiné à extraire des données d'un signal porteur modulé et un moyen de commande pouvant être mis en oeuvre pour utiliser des données extraites par le moyen d'extraction de données afin de valider un paiement pour au moins un produit ou service devant être effectué par le dispositif de communications portable.

19. Dispositif de paiement selon la revendication 18, dans lequel le moyen de commande peut être mis en oeuvre pour utiliser des données extraites par le moyen d'extraction de données afin d'autoriser le moyen de communication à accéder à un emplacement sur le réseau pour valider un paiement devant être effectué.
